(19)

Europäisches
Patentamt
European
Patent Office
Office européen
des brevets

(11)     **EP 3 700 429 B1**

(12)                    **EUROPEAN PATENT SPECIFICATION**

(45) Date of publication and mention
of the grant of the patent:
**04.08.2021 Bulletin 2021/31**

(21) Application number: **18830017.2**

(22) Date of filing: **26.10.2018**

(51) Int Cl.:
**A61B 8/08** (2006.01)

(86) International application number:
**PCT/UA2018/000118**

(87) International publication number:
**WO 2019/083491 (02.05.2019 Gazette 2019/18)**

(54) **METHOD AND APPARATUS FOR ULTRASOUND MEASUREMENT AND IMAGING OF BIOLOGICAL TISSUE ELASTICITY IN REAL TIME**

VERFAHREN UND VORRICHTUNG ZUR ULTRASCHALLMESSUNG UND BILDGEBUNG DER ELASTIZITÄT VON BIOLOGISCHEM GEWEBE IN ECHTZEIT

PROCÉDÉ ET APPAREIL DE MESURE ET D'IMAGERIE PAR ULTRASONS DE L'ÉLASTICITÉ DE TISSUS BIOLOGIQUES EN TEMPS RÉEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2017 UA 201710409**

(43) Date of publication of application:
**02.09.2020 Bulletin 2020/36**

(73) Proprietor: **AO NPF "BIOSS"**
**Moscow, Zelenograd 124489 (RU)**

(72) Inventors:
• **Barannyk, Yevgen Oleksandrovych**
**M. Kharkiv 61099 (UA)**
• **Boichenko, Yurii Petrovych**
**M. Kharkiv 61115 (UA)**

• **Kniazev, Oleksii Valeriiovych**
**M. Kharkiv 61129 (UA)**
• **Linska, Hanna Volodymyrivna**
**M. Kharkiv 61135 (UA)**
• **Marusenko, Anatolii Llarionovych**
**M. Kharkiv 61138 (UA)**
• **Pupchenko, Viktor Ivanovych**
**M. Kharkiv 61143 (UA)**

(74) Representative: **Viering, Jentschura & Partner mbB**
**Patent- und Rechtsanwälte**
**Am Brauhaus 8**
**01099 Dresden (DE)**

(56) References cited:
**WO-A1-2011/064688     US-A1- 2016 345 939**

**Description**

FIELD OF THE INVENTION

The invention relates to ultrasound methods and apparatus of medical diagnostic using ultrasound probing for estimation of the biological tissue elastic properties by determining the shear wave velocity and imaging the tissue elastic properties.

BACKGROUND

Pathological conditions can lead to a change of elasticity of soft biological tissue in comparison with the physiological conditions. As a result, physicians use palpation for estimation of the elasticity and identification of the pathological conditions of tissue. For example, in the case of such diffuse diseases of the liver as fibrosis, cirrhosis and others, the stiffness of the tissue increases and can be evaluated by palpation. However, such evaluation of elastic properties is not quantitative and this limits its diagnostic value.

The method and apparatus for acquiring an image of the shear modulus or Young's modulus of biological tissue combining pulsed ultrasound probing with the simultaneous creation of external static stresses on biological tissue are known (see US5474070). The proposed method comprises creating an external static stresses on the surface of the object of study, assigning a plurality of probing lines, further transmitting a plurality of probing ultrasound pulses into biological tissue along each of the probing lines, receiving a plurality of ultrasound signals from the biological tissue generated in response to the plurality of probing ultrasound pulses, detecting the tissues displacements caused by stresses with the help of response signals, determining the tissue strain on the basis of displacements magnitude and calculation of shear modulus according to the strain and external stress values.

In described method the stress on the surface of biological object may be created by the same ultrasound transducer that performs ultrasound probing. The disadvantage of this method is the low accuracy of measurements, since in real conditions it is impossible to create the homogeneous strain in biological objects, which have usually complex spatial geometry. As a result, the actual local stresses within the biological tissue can differ significantly from the surface ones, even when the last ones are precisely defined. Therefore, at present this method is qualitative (see US9220479) and as a matter of fact, it does not allow quantitative measurements of the biological tissues elastic properties.

For the generation of shear deformations within biological tissues, it is possible to use the acoustic radiation force created by power focusing ultrasound beam of waves (see Sarvazuan A. et al., Shear wave elasticity imaging: A new ultrasonic technology of medical diagnostics, Ultrasound Med. Biol. 1998, 24 (9), 1419-1435). The applying of a pulsed beam of waves in a certain axis of excitation results in a dynamic response of the biological tissue to the pulsed acoustic radiation force, which has the greatest value in the focal area of the beam. As a result, the tissue performs initial shear displacement in the focal area, which leads further to the shear wave excitation and propagation. A response of the biological tissue in the form of excited shear wave, the source of which is the focal area, can be detected, for example, by the ultrasound Doppler method (see Barannik E.A. et al., Doppler ultrasound detection of shear waves remotely induced in tissue phantoms and tissues in vitro. Ultrasonics, 2002, 40 (1-8), 849-852) and other ultrasound methods (see Nightingale K. et al., Shear wave generation using acoustic radiation force: in vivo and ex vivo results, Ultrasound Med. Biol., 2003 , 32 (1), 61-72), which are used to determine tissue displacement. The shear wave propagation velocity is determined by the shear modulus and the biological tissue density; therefore, the determining of the shear wave velocity solves the problem of finding the shear modulus and the tissue Young's modulus.

A large number of methods for measuring and imaging the biological tissue elasticity, in particular, Barannik E. et al., The influence of viscosity on the shear strain remotely induced by focused ultrasound in viscoelastic media, Journ. Acoust. Soc. Am., 2004, 115 (5, Pt.1) 2358-2364, McLaughlin J. et al., Shear wave speed recovery in transient elastography and supersonic imaging using propagating fronts, Inverse problems, 2006, 22, 681-706, US8118744 , US7252004 and UA104530 is based on this principle of combined ultrasound probing and simultaneous remote excitation of shear wave. The "time of flight" method (see Barannik E. et al., The influence of viscosity on the shear strain remotely induced by focused ultrasound in viscoelastic media, Journ. Acoust. Soc. Am., 2004, 115(5 , Pt.1) 2358-2364, McLaughlin J. et al., Shear wave speed recovery in transient elastography and supersonic imaging using propagating fronts, Inverse problems, 2006, 22, 681-706, US8118744), i.e. a method based on the measuring the time of shear wave propagation between the sample volumes, which have different spatial location in the direction perpendicular to the axis of excitation, is well known. In this case, the shear wave propagation velocity can be estimated with dividing the distance between the sample volumes by the time of the wave propagation between them. The calculations are performed on the results of measurements in a plurality of sample volumes, located at the same depth and at different distances from the axis of excitation. The accuracy of such measurements strongly depends on whether the shear wave actually propagates perpendicularly to the axis of excitation. The reason is that the "time of flight" method follows as a matter of fact from the one-dimensional inverse Eikonal equation (see McLaughlin J. et al., Shear wave speed recovery in transient elastography and supersonic imaging using propagating fronts, Inverse problems, 2006, 22, 681-706), which is incapable of taking into account possible changes in the direction of the shear wave propagation. In practice, the same constant direction of propagation can not be achieved even for different parts of the same wave front, as discussed below.

A method and apparatus for ultrasound measurement and imaging of biological tissues elastic properties by means of shear waves (see US7252004) are known. The method includes applying a power ultrasound beam of waves to biological tissue along a predetermined axis to excite shear waves in the biological tissue, assigning a plurality of probing lines, further transmitting a plurality of probing ultrasound pulses along each of the probing directions, receiving a plurality of ultrasound signals from the biological tissue generated in response to the plurality of probing ultrasound pulses, detecting the tissue displacements, which are caused by propagation of shear wave, in a plurality of sample volumes with different spatial location based on the received ultrasound response pulses, finding spectral components of tissues displacements, determining on this basis the spectral components of second order displacement derivatives in both space and time, evaluating by their means the velocity of shear wave propagating through the sample volumes with different spatial location, determining the shear modulus, and imaging the modulus in real time.

The use of the information on the tissue displacements in a plurality of sample volumes, which are located at different depths and different probing lines, in the method allows to evaluate the shear wave velocity directly from the inversion of the Helmholtz two-dimensional wave equation. The latter increases the accuracy of determining the biological tissue elastic parameters, since it takes into account the possibility of shear waves propagation in an arbitrary direction relatively to the axis of excitation.

The disadvantage of this method is the low accuracy of the determining the second derivatives and their spectral components that decreases the signal-to-noise ratio. In addition, the spatial resolution of the method is substantially limited, since the determining the shear wave velocity requires the detecting the tissue displacement data from at least three probing lines and three sample volumes located at different depths. It means that the transverse and longitudinal spatial resolution of this method is equal to $2d_\perp$ and $2d_\parallel$, respectively, where $d_\perp$ - is the distance between the probing lines and $d_\parallel$ - is the distance between the sample volumes located at different depth on a given probing line.

WO 2011/064688 A1 discloses method for ultrasound measurement and visualization of biological tissue elasticity in real time, the method comprising:

> a. applying a power ultrasound beam of waves to biological tissue along a predetermined axis to excite shear waves in the biological tissue,
> b. assigning a plurality of probing lines and transmitting a plurality of probing ultrasound pulses along each of the probing lines,
> c. receiving a plurality of ultrasound signals from the biological tissue generated in response to the plurality of probing ultrasound pulses,
> d. detecting the tissue displacements, which are caused by propagation of shear wave, in a plurality of sample volumes with different spatial location based on the received ultrasound response pulses,
> e. determining at least one parameter of elasticity of biological tissue, including the propagation velocity of shear wave, by determining the velocity of the shear wave front in a direction perpendicular to the axis of excitation,
> f. acquiring an image of at least one parameter of elasticity of biological tissue.

WO 2011/064688 A1 discloses an apparatus for ultrasound measurement and imaging of biological tissue elasticity in real time, the apparatus comprising:

> a. an ultrasound transducer,
> b. a receiver-transmitter,
> c. a tissue displacement processor,
> d. a tissue elasticity module, which comprises a tissue elasticity processor and a calculator of the wave front velocity in a direction perpendicular to the axis of excitation, wherein the input of the calculator is connected to the output of tissue displacement processor and the output of the calculator with the input of the tissue elasticity processor,
> e. a data collection and averaging unit,
> f. a scan converter,
> g. a display monitor.

The prior art discussed above is the closest prior art for the invention.

SUMMARY OF THE INVENTION

The proposed invention is aimed to increase the accuracy and reliability of measurements and to increase the spatial resolution when imaging the biological tissue elasticity parameters in real time.

The problem is solved by the method of ultrasound measurement and imaging of biological tissue elasticity in real time, the method comprising:

applying a power ultrasound beam of waves to biological tissue along a predetermined axis to excite shear waves in the biological tissue,
assigning a plurality of probing lines and transmitting a plurality of probing ultrasound pulses along each of the probing lines,
receiving a plurality of ultrasound signals from the biological tissue generated in response to the plurality of probing ultrasound pulses,
detecting the tissue displacements, which are caused by propagation of shear wave, in a plurality of sample volumes with different spatial location based on the received ultrasound response pulses,
determining at least one parameter of elasticity of biological tissue, including the propagation velocity of shear wave,
acquiring an image of at least one parameter of elasticity of biological tissue,
characterized in that the method comprises:

determining the velocity of the shear wave front in a direction perpendicular to the axis of excitation,
determining the velocity of the wave front along the axis of excitation,
determining a noise level that occurred at the determining of said velocities of the wave front,
determining the shear wave propagation velocity based on the said velocities of the wave front along and perpendicular to the axis of excitation;
acquiring an image of at least one parameter of elasticity of biological tissue based on the determined shear wave propagation velocity and the noise level, and
acquiring noise level image.

In certain embodiments, the method comprises:

applying a sequence of power ultrasound beams of waves to biological tissue to excite shear waves in the biological tissue, and
acquiring the image of at least one parameter of elasticity of biological tissue and the noise level image after each applying in real time.

In certain embodiments, the determining the wave front velocity in a direction perpendicular to the axis of excitation is based on the detecting the wave front time of flight between the sample volumes located at different distances from the axis of excitation.

In certain embodiments, the determining the wave front velocity along the axis of excitation is based on the detecting the wave front time of flight between the sample volumes located at a different depth along the axis of excitation.

In certain embodiments, the acquiring an image of at least one parameter of elasticity of biological tissue based on the determined shear wave propagation velocity and the noise level comprises:

comparing the noise level with the preset value;
if the noise level is not greater than the preset value, at least one parameter of the elasticity of the biological tissue is determined on the basis of determined shear wave propagation velocity or by the weighted averaging with the value of this parameter obtained at previous applying the power ultrasound beam of waves,
if the noise level is greater than the preset value, at least one parameter of the elasticity of the biological tissue is determined on the basis of the value of this parameter obtained at previous applying the power ultrasound beam of waves and/or on the basis of the nearest in the space values of this parameter for which the noise level is not greater than the preset value.

Another object of the invention is an apparatus for ultrasound measurement and imaging of biological tissue elasticity in real time, the apparatus comprising:

ultrasound transducer,
receiver-transmitter,
tissue displacement processor,
tissue elasticity module, which comprises a tissue elasticity processor,
data collection and averaging unit,
scan converter,
display monitor,

wherein the tissue elasticity module, which comprises a tissue elasticity processor, additionally comprises:

calculator of the wave front velocity in a direction perpendicular to the axis of excitation, the input of which is connected to the output of tissue displacement processor, and the first output with the first input of the tissue elasticity processor,

calculator of the wave front velocity along the axis of excitation, the input of which is connected to the output of tissue displacement processor, and the first output with the second input of the tissue elasticity processor,

noise level calculator, the first input of which is connected to the output of tissue displacement processor, the second and third inputs are connected to second outputs of the wave front velocity calculators, and the output to the second input of the data collection and averaging unit,

comparator whose input is connected to the output of the noise level calculator, and the output to the first input of the data collection and averaging unit.

Entering these additional elements and connections to the apparatus allows to implement the proposed method for elasticity ultrasound measurements and thereby to increase the accuracy and reliability of measurements, as well as the resolution at imaging the biological tissue elasticity parameters in real time.

DESCRIPTION OF THE FIGURES

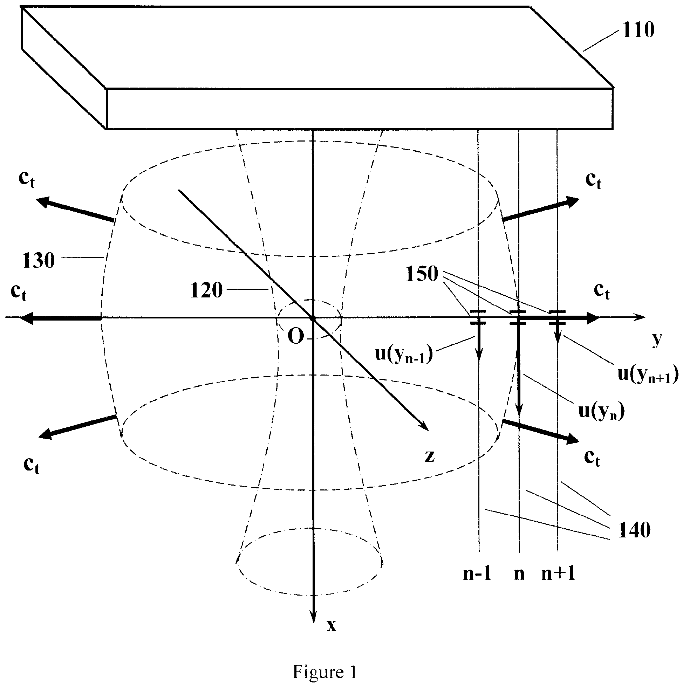

FIG. 1 shows an ultrasound transducer, an axis of excitation $Ox$ in the biological tissue, along which the power ultrasound beam of waves is applying for the shear wave excitation in tissue, a plurality of probing lines in a plane $(x, y)$, numbered by the index $n$ and located at different distances from the axis of excitation, the wave front of the maximum shear wave displacements, which propagates with velocity $c_t$, and the displacement values $u(\vec{r},t)$ at a given time in three sample volumes, which are located on the adjacent probing lines and have the same coordinate $x$, i.e. located at the same depth relatively to the ultrasound transducer.

FIG. 2 shows a computer simulation of the tissue displacements dependence on the time in the sample volumes, which are at the same depth relatively to the ultrasound transducer and located on the adjacent probing lines.

FIG. 3 shows the relative location of the wave front, the two probing lines with the sample volumes, the distance $d_\perp$ between the probing lines, the distance $d_\parallel$ between the sample volumes in the given probing line and the angle $\alpha$ between the wave front and the probing lines.

FIG. 4 shows a diagram of an apparatus for ultrasound measurement and imaging of the biological tissue elasticity in real time.

FIG. 5 shows an image of the ultrasound phantom of biological tissue, when grayscale technique is employed, Young's modulus in the phantom displayed in a color image, and displaying measurement results.

FIG. 6 shows an image of the ultrasound phantom of biological tissue, when grayscale technique is employed, a noise level at measurements within phantom displayed in a color image, and displaying measurement results.

DETAILED DESCRIPTION OF THE INVENTION

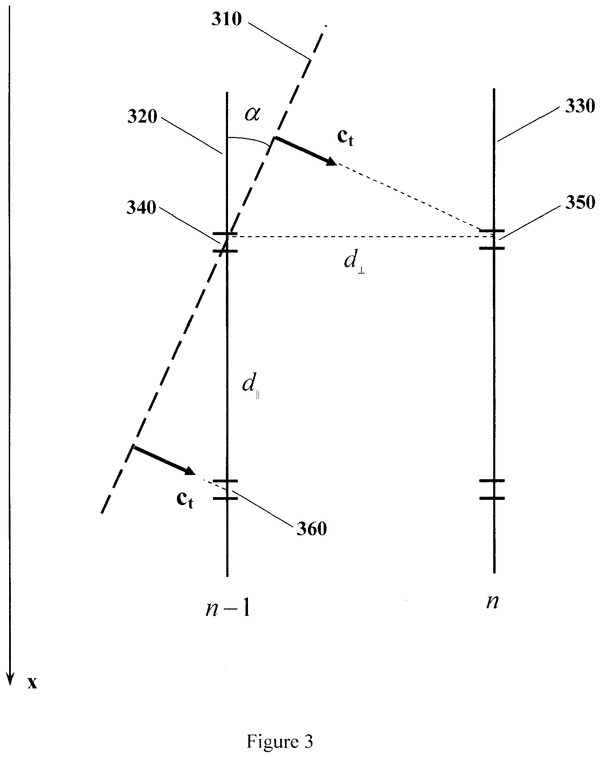

In accordance with the invention, the method of ultrasound measurement and imaging of the biological tissue elasticity in real time comprises applying a power ultrasound beam of waves **120** along a predetermined axis $Ox$ for shear wave excitation in the tissue, as shown in FIG. 1. A plurality of probing lines **140** is assigned and a plurality of probing ultrasound pulses is transmitted along each of the probing lines. A plurality of ultrasound signals from the biological tissue generated in response to the plurality of probing ultrasound pulses is received. By means of response signals, the tissue displacements, which are caused by propagation of shear wave, in a plurality of sample volumes **340**, **350** and **360** with different spatial location are detected, as shown in FIG. 3. On this basis, the wave front propagation velocity **130** in a direction perpendicular to the axis of excitation $Ox$, as well as the wave front propagation velocity along the axis of excitation $Ox$ are determined. The noise level that occurred during the determining the said wave front propagation velocity is determined. The shear wave propagation velocity by means of the determined wave front velocities along and perpendicular to the shear wave axis of excitation is determined. On the basis of the determined shear wave propagation velocity values and the noise level, an image of at least one parameter of the biological tissue elasticity and the noise level image is acquired.

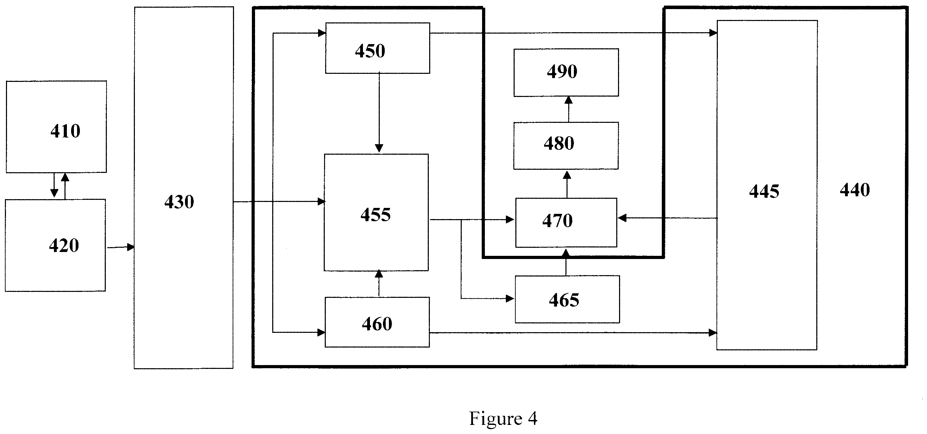

A diagram of elements of an apparatus for ultrasound measurement of the biological tissue elastic properties in real time, as shown in FIG. 4, includes an ultrasound transducer **410**, a receiver-transmitter **420**, a tissue displacement processor **430**, and a tissue elasticity module **440** that contains a soft a tissue elasticity processor **445**, a calculator of the wave front propagation velocity in a direction perpendicular to the axis of excitation **450**, a calculator of the wave front propagation velocity in a direction along the axis of excitation **460**, a noise level calculator **455**, a comparator **465**, a data collection and averaging unit **470**, a scan converter **480**, and a display monitor **490**.

When implementing the proposed method, an apparatus works as follows. The receiver-transmitter **420** generates a pulse signal through which the ultrasound transducer **410** transmits power focusing ultrasound beam of waves that becomes a source of shear waves in the biological tissue. FIG. 1 shows transmitting a power focusing ultrasound beam of waves **120** by the ultrasound transducer **110**. The focal area of such beam with the center at origin of coordinates $O$ is the most effective shear wave source due to the greatest acoustic radiation force. Excited shear wave front **130** is not perfectly cylindrical in all cases, so that the propagation velocity in different parts of the wave front has different directions. In some embodiments of the proposed method, the use of power ultrasound beams of waves with arbitrary spatial configuration of an ultrasound field and, as a consequence, with arbitrary geometry of excited shear wave front is possible. For real-time forming of images a sequence of power focusing ultrasound beam of waves for shear wave excitation in the tissue is generated.

After each applying of power ultrasound beam of waves, the receiver-transmitter **420** generates a pulsed periodic signal, which is converted by the ultrasound transducer **410** into a sequence of probing ultrasound pulses transmitting along each of the assigned probing lines. The ultrasound signals from the biological tissue generated in response to the plurality of probing ultrasound pulses are received by the ultrasound transducer **410** and converted into electrical response signals that arrive to the receiver-transmitter **420**, which amplifies them to the value necessary for further processing in the tissue displacement processor **430**.

Data from the receiver-transmitter **420** to the tissue displacement processor **430** may be received both in the form of radio frequency and low-frequency response signals. In the latter case, the receiver-transmitter **430** performs quadrature demodulation of the radio frequency response signals using the heterodyne complex signal, resulting in low-frequency complex Doppler I-Q response signals in the form of two quadrature components for the probing pulses of each probing lines. Analog-digital signal conversion is also carried out in the receiver-transmitter **420**, and as a result, the discrete response signals from the sample volumes come to the input of the tissue displacement processor **430**.

The above-described method of processing signals in the receiver-transmitter **420** using quadrature demodulation corresponds to a further autocorrelation algorithm for determining displacements (see US4473477 and US4840028) in the tissue displacement processor **430**. In the general case, the calculating of the shear displacement $u(\vec{r},t)$ at time $t$ in a sample volume with a coordinate $\vec{r}$ can be realized in the displacement processor **430** using, for example, the cross-correlation of radio frequency signals, which is also known as a speckle tracking method and is proposed in US5474070 and US7252004, as well as with usage of any other known method described, for example, in US9220479.

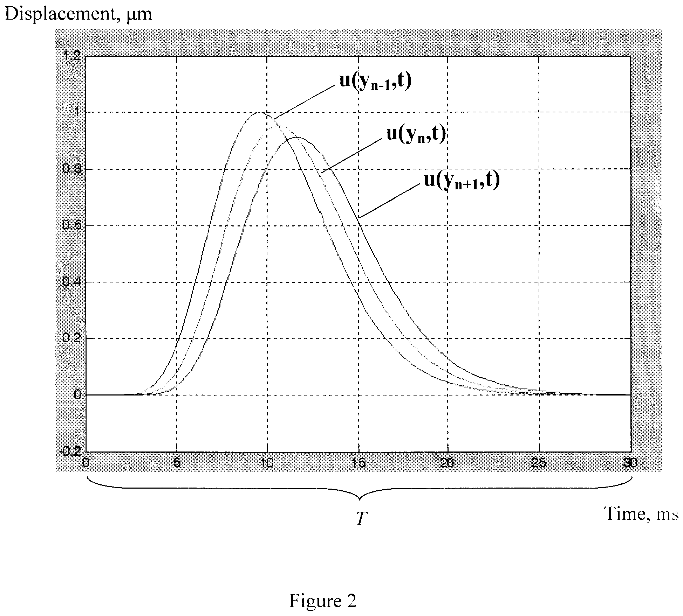

Tissue elasticity module **440** receives further tissue displacement data determined at different times. These data about displacements observed in a predetermined time interval $T$ in sample volumes with different spatial location are stored in the calculator of the wave front propagation velocity in a direction that is perpendicular to the axis of excitation **450**, and in the calculator of the wave front propagation velocity along the axis of excitation **460**. The value of the time interval $T$ is defined by the sample volumes spatial coordinates, the smallest of the possible shear wave propagation velocity and the greatest possible shear wave front time of flight through the sample volume. These conditions provide the detection of all displacements caused by the shear wave propagation in sample volumes, as shown, for example, in FIG. 2 as displacements in three sample volumes **150** on adjacent probing lines **140**.

FIG. 2 shows also the characterized tissue displacements dependence on the time $u(\vec{r},t)$ in the sample volumes **150**, which are located on adjacent probing lines **140** and have the same coordinate $x$, so that they are located at the same depth relatively to the ultrasound transducer. The time of flight $\tau_{\perp}$ of the wave front **310** between two sample volumes **340** and **350** having the same coordinate $x$ and located in adjacent probing lines **320** and **330**, shown in FIG. 3, can be obtained in the calculator **450** using the cross-correlation function as follows:

$$C(\tau) = \int_{0}^{T} u(y_{n-1}, x, t) u(y_{n}, x, t + \tau) dt$$

The time of flight $\tau_{\perp}$ of wave front is equal to those time displacement $\tau$ which makes the maximum cross-correlation function.

The value $\tau_{\perp}$ may also be obtained using the sum of absolute differences (SAD) of displacements, the sum of square differences (SSD) of displacements, the sum of absolute cubic differences (SCD) of displacements, or the sum of the absolute power differences (SPD) of displacements as follows,

$$SAD(\tau) = \int_0^T \left| u(y_{n-1}, x, t) - u(y_n, x, t + \tau) \right| dt$$

$$SSD(\tau) = \int_0^T \left[ u(y_{n-1}, x, t) - u(y_n, x, t + \tau) \right]^2 dt$$

$$SCD(\tau) = \int_0^T \left| u(y_{n-1}, x, t) - u(y_n, x, t + \tau) \right|^3 dt$$

$$SPD(\tau) = \int_0^T \left| u(y_{n-1}, x, t) - u(y_n, x, t + \tau) \right|^p dt$$

where $p$ - is an arbitrary positive number. At determining of the time of flight $\tau_\perp$ with given functions, this value is equal to those time displacement $\tau$ which makes the minimum of each of these functions. As a result, the wave front propagation velocity in direction perpendicular to the axis of excitation may be determined with calculator **450** as the following equation,

$$c_\perp = \frac{d_\perp}{\tau_\perp}.$$

According to FIG. 3, at an angle $\alpha \neq 0$ the same wave front **310** at different times reaches the sample volumes **340** and **360**, located on the $n$ - 1 probing line **320**, but having different coordinates $x$, so that they are located at different depths. Determining the wave front time of flight $\tau_\parallel$ between different sample volumes on a given probing line with calculator **460** is performed in the same way as the time $\tau_\perp$, i.e. with the usage of one of the functions $C(\tau)$, $SAD(\tau)$, $SSD(\tau)$, $SCD(\tau)$ or $SPD(\tau)$. As a result, the calculator **460** determines the wave front velocity along the axis of excitation, which is equal to

$$c_\parallel = \frac{d_\parallel}{\tau_\parallel}.$$

Thus, in order to determine the local wave front propagation velocities perpendicularly and along the axis of excitation, it is sufficient to have the information about the tissue displacement within two sample volumes in adjacent probing lines and in two adjacent sample volumes in a given probing line, respectively. Therefore, the transverse and longitudinal spatial resolution of the proposed method is equal to $d_\perp$ and $d_\parallel$, respectively.

The dimensionless value $\delta_\perp$ of the noise level that occurs at determining the value $c_\perp$ is carried out by the noise level calculator **455**, which receives the data on the value $\tau_\perp$ from the calculator **450** and the data on the tissue displacement at different times from the tissue displacement processor **430**. The fact that when $\tau = \tau_\perp$ function $C(\tau)$ passes through a maximum and functions $SAD(\tau)$, $SSD(\tau)$, $SCD(\tau)$ or $SPD(\tau)$ passes through a minimum means that at this time displacement the tissue displacement curves in different sample volumes coincide most strongly. In reality, the ideal coincidence cannot be achieved due to various physical reasons, which decreases the accuracy of measurements. Firstly, the shape of these curves is distorted due to the natural shear wave attenuation and their cylindrical divergence in propagating wave, which results in decreasing of the wave magnitude in the sample volumes, which are more remote from the shear wave source, as shown in FIG. 2. Secondly, the speckle noise and noises of other nature lead to the curves shape distortion. Thirdly, the shape of displacement curve may change, if the shear wave propagates through the tissue heterogeneities, as well as due to the natural tissue movements.

Thus, the functions value $C(\tau_\perp)$, $SAD(\tau_\perp)$, $SSD(\tau_\perp)$, $SCD(\tau_\perp)$ or $SPD(\tau_\perp)$ objectively reflects all the physical factors and noises that lead to distortion of the shape of displacement curves, decrease the accuracy of determining the value $c_\perp$ and further estimation of the biological tissue elasticity. On the other hand, the absolute value of these functions also depends on the initial amplitude of excited shear waves, which is unknown in advance. Therefore, for the noise level evaluation in the proposed method, functions normalized to the tissue displacement curves power are used. Namely, at calculating the value $c_\perp$ the noise level evaluation can be carried out in accordance with the following equations:

$$\delta_\perp = 1 - \frac{C(\tau_\perp)}{\left\{ \int_0^T u^2(y_{n-1},x,t)dt \int_0^T u^2(y_n,x,t+\tau_\perp)dt \right\}^{\frac{1}{2}}},$$

$$\delta_\perp = \frac{T^{-\frac{1}{2}} SAD(\tau_\perp)}{\left\{ \int_0^T u^2(y_{n-1},x,t)dt \int_0^T u^2(y_n,x,t+\tau_\perp)dt \right\}^{\frac{1}{4}}},$$

$$\delta_\perp = \frac{SSD(\tau_\perp)}{\left\{ \int_0^T u^2(y_{n-1},x,t)dt \int_0^T u^2(y_n,x,t+\tau_\perp)dt \right\}^{\frac{1}{2}}},$$

$$\delta_\perp = \frac{T^{\frac{1}{2}} SCD(\tau_\perp)}{\left\{ \int_0^T u^2(y_{n-1},x,t)dt \int_0^T u^2(y_n,x,t+\tau_\perp)dt \right\}^{\frac{3}{4}}},$$

$$\delta_\perp = \frac{T^{\frac{p}{2}-1} SCD(\tau_\perp)}{\left\{ \int_0^T u^2(y_{n-1},x,t)dt \int_0^T u^2(y_n,x,t+\tau_\perp)dt \right\}^{\frac{p}{4}}}.$$

If the functions $SAD(\tau_\perp)$, $SSD(\tau_\perp)$, $SCD(\tau_\perp)$ or $SPD(\tau_\perp)$ are used for noise level evaluation the normalization to the tissue displacement curves power can be also replaced by the normalization to the cross-correlation function, the calculation of which takes less time.

The dimensionless value $\delta_\parallel$ of the noise level that occurs at determining the value $c_\parallel$ is carried out in a similar way by the noise level calculator **455**, which receives the value $\tau_\parallel$ from the calculator **460**. In this calculator, the noise level is also estimated taking into account both values $\delta_\perp$ and $\delta_\parallel$ according to one of the equations:

$$\delta = \frac{1}{2}(\delta_\perp + \delta_\parallel), \ \delta = \sqrt{\delta_\perp \delta_\parallel}, \ \delta = \max\{\delta_\perp, \delta_\parallel\}, \ \delta = \min\{\delta_\perp, \delta_\parallel\}.$$

Determined level of the noise comes to the comparator **465** and data collection and averaging unit **470**, which forms the dataset necessary for acquiring the noise level image. The same unit also defines the average noise level as well as the minimum and maximum values in the selected region of interest. A noise level image is formed in the scan converter **480** with further displaying on display monitor **490**.

Data about the determined velocity values $c_\perp$ and $c_\parallel$ from the calculators **450** and **460** come to the soft tissue elasticity module **445**, which determines the velocity of the shear wave. In some embodiments, the probing lines can be parallel to the axis of excitation, as shown in FIG. 1 and FIG. 3. As can be seen from FIG. 3, in this case the time of flight of wave front between the sample volume **340**, having a coordinate $x$ at the probing line **320** with number $n - 1$, and the sample volume **350**, having the same coordinate $x$ at the probing line **330** with number $n$, is equal to

$$\tau_\perp = \frac{d_\perp \cos \alpha}{c_t}.$$

Similarly, the wave front time of flight between the sample volumes **340** and **360** located at the probing lines **320** is equal to

$$\tau_\parallel = \frac{d_\parallel \sin \alpha}{c_t}.$$

It means that the wave front velocity in the direction perpendicular to the axis of excitation and along the axis of excitation are determined by the shear waves velocity and are described by the formulas

$$c_\perp = \frac{c_t}{\cos \alpha}, \qquad c_\parallel = \frac{c_t}{\sin \alpha}.$$

Both of the presented velocities in the general case can have an arbitrary sign, depending on the direction of wave propagation and the local value of angle $\alpha(x, y)$, and their absolute values satisfy the inequality:

$$\left| c_\perp (x, y) \right|, \left| c_\parallel (x, y) \right| \geq c_t (x, y).$$

In an example shown in FIG.3 $x$ and $y$ are the coordinates of the sample volume **340**. According to obtained wave front velocities the calculator **445** calculates the absolute value of the shear wave local velocity, which does not depend on the value of the angle $\alpha(x, y)$:

$$c_t (x, y) = \left| \frac{c_\perp (x, y) c_\parallel (x, y)}{\sqrt{c_\perp^2 (x, y) + c_\parallel^2 (x, y)}} \right|.$$

The shear wave velocity and the shear modulus $\mu$ have the following relationship

$$\mu(x, y) = \rho(x, y) c_t^2 (x, y),$$

where $\rho(x, y)$- is the local biological tissue density. That is why the calculator **445** also determines the shear modulus and the Young's modulus $E(x, y)$, which for biological tissues have the following relationship

$$E(x, y) = 3\mu(x, y).$$

The determined value of at least one biological tissue elasticity parameter, which includes at least one of shear wave propagation velocity, shear elasticity modulus, and Young's modulus, comes to the data collection and averaging unit **470**, which forms the dataset necessary for further formation of two-dimensional image of this parameter in scan converter **480**.

The same block receives from the comparator **465** the results of a preset value $\delta_{th}$ comparing with noise level that occurred at determining elastic parameters. If the noise level is not greater than the preset value, at least one biological tissue elasticity parameter is determined on the basis of determined shear wave propagation velocity. In some embodiments, a weighted averaging with the value of this parameter obtained at previous applying the power ultrasound beam of waves can be used. If the noise level is greater than the preset value, the value of at least one parameter of the biological tissue elasticity is determined on the basis of the value obtained at previous applying the power ultrasound beam of waves and on the basis of the nearest in the space values of this parameter for which the noise level is not greater than the preset value. In some embodiments, this parameter of the elasticity of the biological tissue can be determined only on the basis of the nearest in the space values of this parameter for which the noise level is not greater than the preset value. After formation the dataset necessary for acquiring an image of at least one biological tissue elasticity parameter, the data collection and averaging unit **470** also defines an average of this parameter, the mean square deviation, as well as the minimum and maximum values in the selected region of interest. The image of at least one biological tissue elasticity parameter is formed in the scan converter **480**, with further displaying on the display monitor **490**.

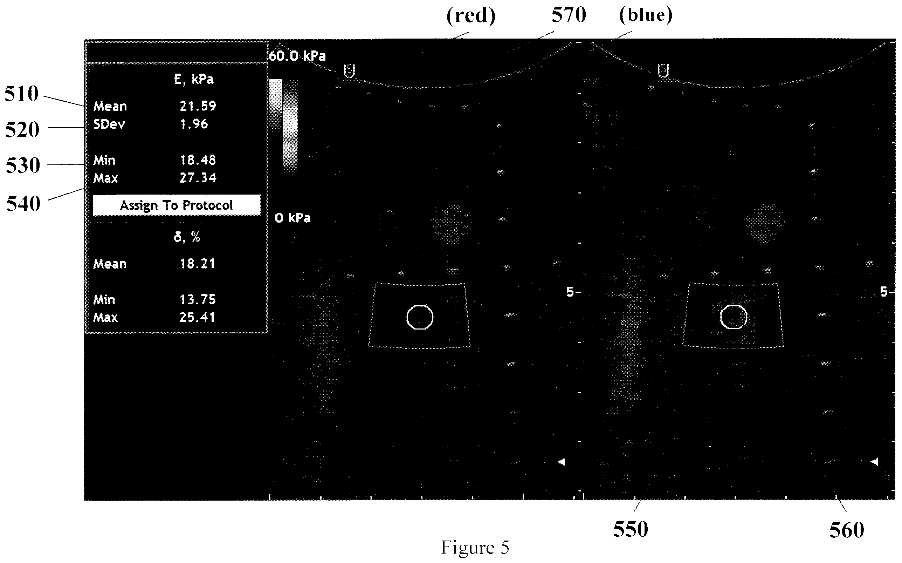

Finally, the display monitor **490**, which receives data from the scan converter **480**, carries out an ultrasound measurement results indication of at least one biological tissue elasticity parameter in real time in the form of an average value **510** of this parameter, a mean deviation **520**, and a minimum **530** and a maximum **540** values in the selected region of interest **550**, as shown in FIG. 5 for the measurement results of the Young's modulus. At the same time, the display monitor **490** images the selected biological tissue elasticity parameter **560**. Both a color coding technique and a grayscale technique may be employed to present the image of at least one biological tissue elasticity parameter. In some embodiments of the method, the Young's modulus is displayed in a color red-blue image **570**, in which a relatively high value of $E \cong 60$ kPa is coded using a red color while a low value is coded using a blue. The choice of the value range at color-coding of the biological tissue elasticity parameter depends mainly on a type of one or another biological tissue studied with apparatus.

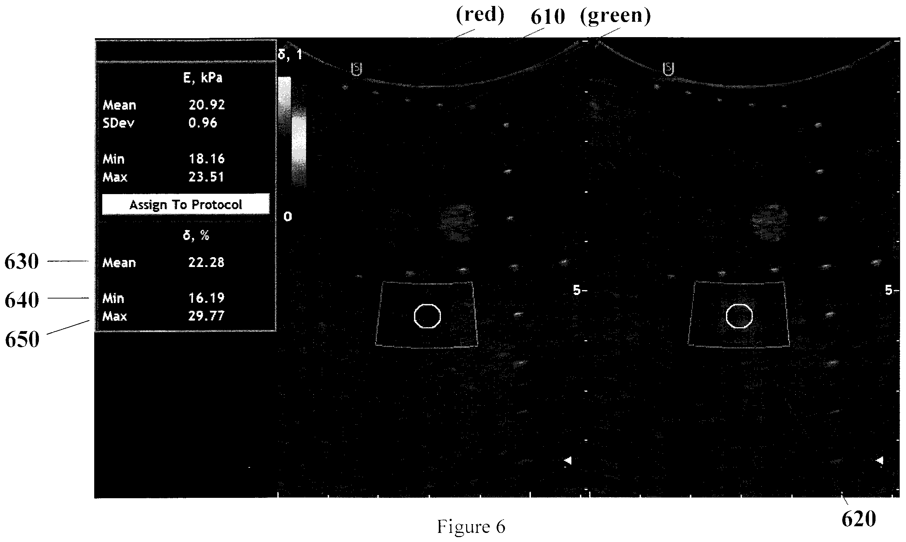

Both a color coding technique and a grayscale technique may be employed to present the image of the noise level. FIG. 6 shows a color-coding **610** of the noise level image **620**, in which a high value of $\delta \cong 1$ ($\cong 100\%$) is coded using red and violet colors, while a low value is coded using a green color. The noise level range $\delta \le 1$ at color-coding and grayscale-coding of images is selected in this embodiment due to the fact that at $\delta > 1$ the obtained value of at least one biological tissue elasticity parameter is unreliable deliberately. The real-time indication of the average noise level **630**, as well as the minimum **640** and the maximum **650** values in the selected region of interest is performed simultaneously with imaging of the Young's modulus measurement results. Providing the noise level image and indication of its magnitude in real time increases the feasibility of results obtained at real-time measurement.

EXAMPLE

The claimed method and apparatus for ultrasound measurement of biological tissue elasticity in real time may be realized, for example, using the XILINX Spartan-6 XC6SLX45 field-programmable gate array (FPGA), static memory (SRAM) chips and a personal computer that can perform all required measurements and calculations in real time using appropriate program products.

A typical plurality of probing lines does not exceed 64, the maximum number of sample volumes on each probing line does not exceed 128 and therefore the total number of sample volumes on all probing lines does not exceed 8192.

The maximum pulse repetition frequency of probing pulses does not exceed 10 kHz and a time of shear wave propagation in biological tissues does not exceed as rule 30 ms. Hence, the maximum number of displacement curve discrete values obtained for one sample volume does not exceed 300. Thus, the total number of discrete values for all sample volumes does not exceed 2457600.

The number of multiplication and addition operations for the implementation of the proposed method necessary for determining tissue displacement and at least one biological tissue elasticity parameter in one sample volume does not exceed $10^5$. Therefore, at a typical frame rate, which does not exceed 5 frames per second, the total number of multiplication and addition operations does not exceed $5 \cdot 10^9$ operations per second.

FPGA XC6SLX45 has 58 multiplier-accumulator modules capable of operating at 300 MHz, which allows up to $17 \cdot 10^9$ multiplication and addition operations per second and 27000 logic modules that allow up to 840 units to perform arithmetic or logic operations with a 32-bit numbers up to 200 MHz, resulting in a total number of operations reaching $168 \cdot 10^9$ arithmetic or logical operations per second, which is much greater than one needed to implement the proposed method in real time. At that the FPGA has a memory of 2088 kb.

The 8-megabyte SRAM chips allow collecting the data about the tissue displacement in all 8192 sample volumes.

Taking into account the peak performance, the said FPGA allows to implement the tissue displacement processor **430**, the tissue elasticity module **440**, comprising the calculator of the shear wave propagation front velocity in a direction perpendicular to the axis of excitation **450**, the calculator of the shear wave propagation front velocity along the axis of excitation **460**, the noise level calculator **455** and the comparator **465** in real time.

The data collection and averaging unit **470**, the scan converter **480**, and the display monitor **490** can be implemented using computer readable program codes for personal computer. In order to transfer data from FPGA to personal computer, it is possible to use a USB 2.0 communication channel with a peak data rate of up to 480 Mbps, which allows real-time transmitting of all data on at least one biological tissue elasticity parameter and noise level, which occurred at their determining.

**Claims**

1. A method for ultrasound measurement and visualization of biological tissue elasticity in real time, the method comprising:

   applying a power ultrasound beam of waves (120) to biological tissue along a predetermined axis to excite shear waves in the biological tissue,
   assigning a plurality of probing lines (140) and transmitting a plurality of probing ultrasound pulses along each of the probing lines,
   receiving a plurality of ultrasound signals from the biological tissue generated in response to the plurality of probing ultrasound pulses,
   detecting the tissue displacements, which are caused by propagation of shear wave, in a plurality of sample volumes with different spatial location based on the received ultrasound response pulses,
   determining at least one parameter of elasticity of biological tissue, including the propagation velocity of shear wave,
   acquiring an image of at least one parameter of elasticity of biological tissue,
   determining the velocity of the shear wave front in a direction perpendicular to the axis of excitation,
   determining the velocity of the wave front along the axis of excitation,
   determining a noise level that occurred at the determining of said velocities of the wave front,
   determining the shear wave propagation velocity based on the said velocities of the wave front along and perpendicular to the axis of excitation;
   acquiring an image of at least one parameter of elasticity of biological tissue based on the determined shear wave propagation velocity and the noise level, and
   acquiring noise level image.

2. A method according to claim 1, wherein the method comprises:

   applying a sequence of power ultrasound beams of waves to biological tissue to excite shear waves in the biological tissue, and
   acquiring the image of at least one parameter of elasticity of biological tissue and the noise level image after each applying in real time.

3. A method according to claim 1, wherein the determining the wave front velocity in a direction perpendicular to the axis of excitation is based on the detecting the wave front time of flight between the sample volumes located at different distances from the axis of excitation.

4. A method according to claim 1, wherein the determining the wave front velocity along the axis of excitation is based on the detecting the wave front time of flight between the sample volumes located at a different depth along the axis of excitation.

5. A method according to claim 1, wherein the acquiring an image of at least one parameter of elasticity of biological tissue based on the determined shear wave propagation velocity and the noise level comprises:

   comparing the noise level with the preset value;
   if the noise level is not greater than the preset value, at least one parameter of the elasticity of the biological tissue is determined on the basis of determined shear wave propagation velocity or by the weighted averaging with the value of this parameter obtained at previous applying the power ultrasound beam of waves,
   if the noise level is greater than the preset value, at least one parameter of the elasticity of the biological tissue is determined on the basis of the value of this parameter obtained at previous applying the power ultrasound beam of waves and/or on the basis of the nearest in the space values of this parameter for which the noise level is not greater than the preset value.

6. An apparatus for ultrasound measurement and imaging of biological tissue elasticity in real time, the apparatus comprising:

ultrasound transducer (410), receiver-transmitter (420), tissue displacement processor (430), tissue elasticity module (440), which comprises a tissue elasticity processor (445), data collection and averaging unit (470), scan converter (480), display monitor,
wherein the tissue elasticity module, which comprises a tissue elasticity processor, additionally comprises:

calculator of the wave front velocity in a direction perpendicular to the axis of excitation (450), the input of which is connected to the output of tissue displacement processor, and the first output with the first input of the tissue elasticity processor,
calculator of the wave front velocity along the axis of excitation (460), the input of which is connected to the output of tissue displacement processor, and the first output with the second input of the tissue elasticity processor,
noise level calculator (455), the first input of which is connected to the output of tissue displacement processor, the second and third inputs are connected to second outputs of the wave front velocity calculators, and the output to the second input of the data collection and averaging unit,
comparator (465) whose input is connected to the output of the noise level calculator, and the output to the first input of the data collection and averaging unit.

**Patentansprüche**

1. Ein Verfahren zur Ultraschallmessung und Visualisierung der Elastizität von biologischen Geweben in Echtzeit, das beinhaltet:

Strahlung mit einem starken Ultraschallwellenstrahl (120) in das biologische Gewebe in einer vordefinierten Richtung für die Anregung im biologischen Gewebe von Scherwellen,
Einstellen einer Vielzahl von Schallrichtungen (140) und Emission von sondierenden Ultraschallimpulsen entlang einer Vielzahl von Schallrichtungen,
Empfang einer Vielzahl von Ultraschallsignalen der Reaktion des biologischen Gewebes auf eine Vielzahl von sondierenden Ultraschallpulsen,
Bestimmung der Gewebebewegung aufgrund der Ausbreitung von Scherwellen unter Verwendung von Antwortsignalen, in einer Vielzahl von Messvolumen mit unterschiedlichen räumlichen Lokalisierungen,
Berechnung von mindestens einem Parameter der Elastizität des biologischen Gewebes, einschließlich der Geschwindigkeit der Ausbreitung von Scherwellen,
Bildgebung von mindestens einem Parameter der Elastizität des biologischen Gewebes
Messung der Geschwindigkeit der Schubwellenfront in der Richtung, die senkrecht zur Erregungsrichtung ist,
Messung der Geschwindigkeit der Wellenfront entlang der Erregungsrichtung,
Bestimmung des Lärmpegels, der bei der Messung der angegebenen Wellengeschwindigkeiten aufgetreten ist,
Bestimmung der Geschwindigkeit der Scherwellen mit Hilfe der gefundenen Geschwindigkeiten der Wellenfront entlang und senkrecht zur Erregungsrichtung;
Bildgebung von mindestens einem Parameter der Elastizität des biologischen Gewebes auf der Grundlage der gefundenen Werte der Geschwindigkeit der Ausbreitung der Scherwellen und des Lärmpegels, und
Bildgebung von Lärmpegel

2. Verfahren nach Anspruch 1, worin das Verfahren umfasst:

Emission einer Sequenz von starken Ultraschallwellenstrahlen in biologisches Gewebe, um Scherwellen darin zu stimulieren, und
Bildgebung von mindestens einem Parameter der Elastizität des biologischen Gewebes und Bildgebung von Lärmpegel in Echtzeit nach jeder Strahlung.

3. Verfahren nach Anspruch 1, worin die Messung der Geschwindigkeit der Wellenfront in einer zur Erregungsrichtung senkrechten Richtung, auf der Messung der Laufzeit der Wellenfront zwischen den Messvolumina basiert, die sich in unterschiedlichen Abständen von der Erregungsrichtung befinden.

**4.** Verfahren nach Anspruch 1, worin die Messung der Geschwindigkeit der Wellenfront in einer Erregungsrichtung, auf der Laufzeitmessung von Wellenfront zwischen den Messvolumina basiert, die sich in unterschiedlichen Tiefen entlang der Erregungsrichtung befinden.

**5.** Verfahren nach Anspruch 1, worin die Bildgebung von mindestens einem Parameter der Elastizität des biologischen Gewebes auf der Grundlage einer Vielzahl der gefundenen Werte der Geschwindigkeit der Ausbreitung von Scherwellen und des Lärmpegels umfasst:

Vergleich des Lärmpegels mit einem vordefinierten Wert,
wenn der Lärmpegel nicht größer als der voreingestellte Wert ist, so wird mindestens ein Parameter der Elastizität des biologischen Gewebes auf der Grundlage der gefundenen Geschwindigkeit der Ausbreitung von Scherwellen oder Gewichtsmittelung bestimmt, mit dem Wert dieses Parameters, der bei der vorherigen Strahlung eines starken Ultraschallwellenstrahls erhalten wurde,
wenn der Lärmpegel nicht größer als der voreingestellte Wert ist, wird mindestens ein Parameter der Elastizität von biologischen Gewebe basierend auf einem Wert dieses Parameters bestimmt, der bei der vorherigen Strahlung eines starken Ultraschallwellenstrahls und/oder basierend auf dem Wert der nächsten Werte im Raum dieses Parameters erhalten wurde, für den der Lärmpegel nicht größer als der voreingestellte Wert ist.

**6.** Ein Gerät zur Ultraschallmessung und Visualisierung der Elastizität biologischer Gewebe in Echtzeit, das beinhaltet:

Ultraschallwandler (410),
Sende -und Empfangsgerät (420),
Gewebe-Bewegungsrechner (430),
Einheit für die Berechnung der Gewebelastizität (440), die den Gewebelastizitätsberechner (445) enthält,
Einheit für die Datenakkumulation und -Mittelung (470),
Bildkonverter (480),
Informationsdisplay,
worin Einheit für die Berechnung der Gewebelastizität, die den Gewebelastizitätsberechner enthält, zusätzlich enthält:

den Rechner der Geschwindigkeit der Wellenfront in einer zur Erregungsrichtung senkrechten Richtung (450), dessen Eingang mit dem Ausgang des Gewebelastizitätsberechners, und der erste Ausgang mit dem ersten Eingang des Gewebelastizitätsberechners verbunden sind,
den Rechner der Geschwindigkeit der Wellenfront in Erregungsrichtung (460), dessen Eingang mit dem Ausgang des Gewebelastizitätsberechners verbunden ist, und der erste Ausgang mit dem zweiten Eingang des Gewebelastizitätsberechners verbunden ist,
der Lärmpegel-Rechner (455), dessen erster Eingang mit dem Ausgang des Gewebe-Bewegungsrechners verbunden ist, die zweiten und dritten Eingänge mit anderen Ausgängen der Wellenfront-Geschwindigkeitsrechner verbunden sind, und der Ausgang - mit dem zweiten Eingang der Einheit für die Datenakkumulation und -Mittelung verbunden ist,

den Komparator (465), dessen Eingang mit dem Ausgang des Lärmpegel-Rechners verbunden ist, und der Ausgang - mit dem ersten Eingang der Einheit für die Datenakkumulation und -Mittelung verbunden ist.

**Revendications**

**1.** Procédé de mesure par ultrasons et d'imagerie de l'élasticité des tissus biologiques en temps réel, qui comprend:

le rayonnement dans le tissu biologique d'un faisceau d'ondes ultrasonores puissant (120) dans une direction prédéterminée pour exciter les ondes de cisaillement dans le tissu biologique,
la définition d'une pluralité de directions de détection (140) et le rayonnement le long de chacune des directions d'une pluralité d'impulsions ultrasonores de détection,
la réception d'une pluralité de signaux ultrasonores de réponse du tissu biologique à une pluralité d'impulsions ultrasonores de détection,
la détermination des signaux de réponse du déplacement tissulaire dû à la propagation des ondes de cisaillement dans de nombreux volumes de mesure avec de différentes localisations spatiales,
le calcul d'au moins d'un paramètre d'élasticité du tissu biologique, y compris la vitesse de propagation des ondes de cisaillement,

l'obtention d'une image d'au moins d'un paramètre d'élasticité du tissu biologique,

la mesure de la vitesse du front d'onde de cisaillement dans une direction perpendiculaire à la direction d'excitation,

la mesure de la vitesse du front d'ondes le long de la direction d'excitation,

la détermination du niveau de bruit qui s'est produit lors de la mesure des vitesses spécifiées du front d'ondes,

la détermination de la vitesse des ondes de cisaillement à l'aide des vitesses de front d'ondes trouvées le long et perpendiculairement à la direction d'excitation ;

l'obtention d'une image d'au moins d'un paramètre d'élasticité du tissu biologique sur la base des valeurs trouvées de la vitesse de propagation des ondes de cisaillement et du niveau de bruit, et

l'obtention d'une image du niveau de bruit.

2. Procédé selon la revendication 1, dans lequel le procédé comprend :

l'émission d'une séquence de faisceaux d'ondes ultrasonores puissants dans le tissu biologique pour exciter les ondes de cisaillement, et

l'obtention d'une image d'au moins d'un paramètre d'élasticité du tissu biologique et d'une image en temps réel du niveau de bruit après chaque émission.

3. Procédé selon la revendication 1, dans lequel la mesure de la vitesse du front d'ondes dans une direction perpendiculaire à la direction d'excitation est basée sur la mesure du temps de passage du front d'ondes entre les volumes de mesure situés à une distance différente de la direction d'excitation.

4. Procédé selon la revendication 1, dans lequel la mesure de la vitesse du front d'ondes dans la direction d'excitation est basée sur la mesure du temps de passage du front d'ondes entre les volumes de mesure situés à différentes profondeurs le long de la direction d'excitation.

5. Procédé selon la revendication 1, dans lequel l'obtention d'une image d'au moins d'un paramètre d'élasticité du tissu biologique sur la base d'un ensemble de valeurs trouvées de la vitesse de propagation des ondes de cisaillement et du niveau de bruit comprend :

la comparaison du niveau sonore avec une valeur prédéfinie,

si le niveau de bruit n'est pas supérieur à une valeur prédéterminée, alors au moins un paramètre d'élasticité du tissu biologique est déterminé en fonction de la vitesse de propagation des ondes de cisaillement trouvée ou en fonction de la moyenne de poids avec la valeur de ce paramètre obtenue lors de l'émission précédente d'un faisceau d'ondes ultrasonores puissant,

si le niveau de bruit est supérieur à une valeur prédéterminée, alors au moins un paramètre d'élasticité du tissu biologique est déterminé sur la base de la valeur de ce paramètre obtenu lors de la précédente rayonnement ultra-sonique puissant faisceau d'ondes et/ou sur la base des valeurs les plus proches dans l'espace des valeurs de ce paramètre, pour lesquels le niveau de bruit n'est pas supérieur à une valeur prédéterminée.

6. Dispositif de mesure et d'imagerie par ultrasons de l'élasticité des tissus biologiques en temps réel, qui comprend :

un transducteur à ultrasons (410),

l'émetteur-récepteur (420),

le calculateur du déplacement tissulaire (430),

l'unité de calcul d'élasticité tissulaire (440) contenant un calculateur d'élasticité tissulaire (445),

l'unité d'accumulation et de calcul de la moyenne des données (470),

le convertisseur d'image (480),

l'unité de visualisation,

dans lequel le bloc de calcul d'élasticité tissulaire contenant le calculateur d'élasticité tissulaire contient en outre :

le calculateur de vitesse de front d'ondes dans une direction perpendiculaire à la direction d'excitation (450), dont l'entrée est connectée à la sortie du calculateur du déplacement tissulaire et la première sortie à la première entrée du calculateur d'élasticité tissulaire,

le calculateur de vitesse de front d'ondes le long de la direction d'excitation (460), dont l'entrée est connectée à la sortie du calculateur du déplacement tissulaire et la première sortie à la deuxième entrée du calculateur d'élasticité tissulaire,

le calculateur de niveau de bruit (455) dont la première entrée est connectée à la sortie du calculateur de déplacement tissulaire, les deuxième et troisième entrées sont reliées aux autres sorties des calculateurs de vitesse de front d'ondes, et la sortie est connectée à la deuxième entrée de l'unité d'accumulation et de calcul de la moyenne des données,

comparateur (465) dont l'entrée est connectée à la sortie du calculateur de niveau de bruit et la sortie est connectée à la première entrée de l'unité d'accumulation et de calcul de la moyenne des données.

Figure 1

Displacement, μm

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5474070 A
- US 9220479 B
- US 8118744 B
- US 7252004 B
- UA 104530
- WO 2011064688 A1
- US 4473477 A
- US 4840028 A

**Non-patent literature cited in the description**

- **SARVAZUAN A et al.** Shear wave elasticity imaging: A new ultrasonic technology of medical diagnostics. *Ultrasound Med. Biol.,* 1998, vol. 24 (9), 1419-1435
- **BARANNIK E.A. et al.** Doppler ultrasound detection of shear waves remotely induced in tissue phantoms and tissues in vitro. *Ultrasonics,* 2002, vol. 40 (1-8), 849-852
- **NIGHTINGALE K. et al.** Shear wave generation using acoustic radiation force: in vivo and ex vivo results. *Ultrasound Med. Biol,* 2003, vol. 32 (1), 61-72
- **BARANNIK E. et al.** The influence of viscosity on the shear strain remotely induced by focused ultrasound in viscoelastic media. *Journ. Acoust. Soc. Am.,* 2004, vol. 115 (5), 2358-2364
- **MCLAUGHLIN J. et al.** Shear wave speed recovery in transient elastography and supersonic imaging using propagating fronts. *Inverse problems,* 2006, vol. 22, 681-706